# FASCICULE DE BREVET EUROPEEN

(11) **EP 2 597 135 B1**
(45) Date de publication et mention de la délivrance du brevet: **11.06.2014**
(21) Numéro de dépôt: 12290350.3
(22) Date de dépôt: 16.10.2012
(51) Int. Cl.: C10G 11/18, C10G 45/50, C10G 69/04, C10G 50/00, C10G 67/06, C10G 69/12

(54) **Procédé de production de distillat moyen à partir d'une charge lourde conventionnelle incluant une étape d'hydrogénation selective de la coupe HCO EX FCC**
Herstellungsverfahren von Mitteldestillaten aus einer konventionellen schweren Charge, einschließlich einer selektiven Hydrierphase des HCO-EX-FCC-Verschnitts
Method for generating middle distillates from a conventional heavy feedstock including a step of selective hydrogenation of the HCO EX FCC cut

(30) Priorité: 24.11.2011 FR 1103592
(43) Date de publication de la demande: 29.05.2013
(73) Titulaire: IFP Energies nouvelles, 92852 Rueil-Malmaison Cedex (FR)
(72) Inventeur: Feugnet, Frédéric, 69004 Lyon (FR); Hudebine, Damien, 69007 Lyon (FR); Roux, Romain, 92500 Rueil-Malmaison (FR)

(56) Documents cités:
- EP-A1- 0 432 235
- EP-A2- 1 050 572
- FR-A1- 2 769 635

## Description

### DOMAINE DE L'INVENTION

La présente invention concerne un procédé de conversion d'une charge hydrocarbonée lourde présentant une sélectivité améliorée en distillat moyen. Plus précisément, le procédé selon la présente invention permet de coproduire de l'essence avec un rendement réduit, et d'améliorer la production de distillat moyen d'au moins 2% poids par rapport à la charge, ce qui au regard des tonnages mise en oeuvre dans le procédé est très significatif.

Historiquement les unités de craquage catalytique connues sous le sigle FCC, (abréviation de la terminologie anglo-saxonne "Fluid Catalytic Cracking", qui signifie Craquage Catalytique en lit Fluidisé), sont optimisées en vue de la production de produits légers; gaz liquéfiés (ou GPL), oléfines légères, et essence, afin de répondre soit au marché des polymères, soit aux besoins de la consommation d'essence du parc automobile.

Dans ce type de fonctionnement, la production de bases gazoles demeure limitée.

A l'heure actuelle, étant donnée la forte progression de la diésélisation du parc automobile, la demande en produits de type gazoles se trouve largement accrue. Par conséquent, il devient de plus en plus nécessaire d'orienter la production des raffineries vers la production de bases gazoles et de limiter la production d'essence. Les unités de FCC, présentes dans près d'une raffinerie sur deux, étant d'une part la source principale d'essence, et d'autre part une source importante d'oléfines légères, il est impératif de pouvoir les convertir en unités favorisant la production de gazoles. C'est cette tendance que l'homme du métier résume sous l'appellation FCC à marche "maxi LCO", LCO désignant ici la coupe distillat moyen, c'est çà dire une coupe d'intervalle de distillation compris entre 220°C et 360°C.

Le procédé selon la présente invention permet donc 1) d'améliorer la production de bases gazoles dans les unités de craquage catalytique en lit fluidisé, 2) de limiter la production de coupe lourde faiblement valorisable, mais aussi 3) de limiter la production d'essence, cette coupe étant non recherchée dans le schéma maxi LCO. La présente invention consiste essentiellement dans l'enchaînement d'une unité de FCC avec une ou plusieurs unités d'hydrogénation sélective de la coupe distillat lourd (HCO) produite dans le FCC, ou toute autre coupe riche en composés triaromatiques provenant par exemple des unités de viscoréduction, de Coker, de l'unité dite "Hoil" ou encore la coupe Pygas de l'unité de vapocraquage.

Cette coupe distillat lourd est hydrotraitée sélectivement afin de minimiser la proportion de triaromatiques, tout en maximisant le ratio diaromatiques sur monoaromatiques. Elle est ensuite recyclée dans la zone réactionnelle du FCC afin d'augmenter significativement le rendement en distillat moyen (LCO), mais aussi la sélectivité de cette coupe par rapport à l'essence tout en limitant la production de coke additionnelle.

La coupe dite distillat moyen (LCO) a dans le contexte de la présente invention un intervalle de distillation compris entre 220°C et 360°C.

Le procédé FCC permet de convertir des charges hydrocarbonées lourdes, dont la température d'ébullition initiale est généralement supérieure à 340°C en des fractions hydrocarbonées plus légères, notamment une coupe essence, par craquage des molécules de la charge lourde en présence d'un catalyseur acide. Le FCC produit également des Gaz de Pétrole Liquéfiés (GPL) en quantité importante avec des teneurs en oléfines élevées.

Le procédé objet de la présente invention peut être globalement présenté comme un procédé de production de distillat moyen avec amélioration de la sélectivité distillat moyen sur essence.

La présente invention fait appel à une unité de craquage catalytique suivie d'une ou plusieurs unités d'hydrogénation sélective de la coupe distillat lourd d'intervalle de distillation comprise entre 320°C et 490°C et composée majoritairement de triaromatiques. Cette coupe est habituellement notée (HCO), appellation que nous conserverons dans la suite du texte.

L'unité d'hydrogénation sélective peut également traiter toute autre coupe riche en composés triaromatiques provenant par exemple des unités de viscoréduction, de Coker, de l'unité dite Hoil, ou encore la coupe Pygas de l'unité de vapocraquage.

Le procédé selon l'invention consiste essentiellement dans l'enchaînement de l'unité de craquage catalytique et d'une ou plusieurs unités d'hydrotraitement traitant de façon sélective la coupe HCO, avec recyclage de la coupe HCO hydrotraitée à l'unité de craquage catalytique, ainsi que dans la détermination fine des conditions opératoires de l'hydrotraitement afin de transformer de manière sélective les triaromatiques de la charge de l'unité en diaromatiques, tout en maximisant le ratio diaromatiques sur monoaromatiques. Recyclée dans la zone réactionnelle du FCC, la coupe HCO hydrotraitée sélectivement permet d'améliorer très significativement la sélectivité en distillat moyen du procédé, ainsi que de limiter la production additionnelle d'essence et de coke.

Dans le cas de plusieurs unités d'hydrogénation sélective associées à l'unité de craquage catalytique, ces dernières pourront être agencées en série ou en parallèle.

### EXAMEN DE L'ART ANTERIEUR

L'art antérieur enseigne le recyclage de la coupe dite distillat lourd (HCO) vers la zone réactionnelle du FCC, mais pas le recyclage de ladite coupe hydrotraitée sélectivement en vue de maximiser la formation de distillat moyen. Une différence essentielle de la présente invention par rapport au procédé de l'art antérieur cités ci dessus, tient précisément au caractère sélectif de l'hydrogénation et à la détermination fine de ses conditions opératoires.

Le brevet FR 10/04.585 décrit un procédé de conversion d'une charge lourde permettant d'améliorer la sélectivité en distillat moyen en faisant appel à une unité de craquage catalytique suivie d'une ou plusieurs unités d'oligomérisation d'oléfines permettant de produire préférentiellement une coupe distillat moyen additionnelle. La présente invention consiste en l'enchaînement d'une unité de craquage catalytique (FCC) et d'une ou plusieurs unités d'hydrogénation(s) sélective(s) de distillat lourd permettant d'améliorer significativement la production de distillat moyen et en même temps d'améliorer la sélectivité distillat moyen sur essence tout en limitant la formation de coke additionnel.

FR 2 769 635 décrit un procédé comprenant une étape FCC suivie d'une étape d'hydrotraitement.

### DESCRIPTION SOMMAIRE DES FIGURES

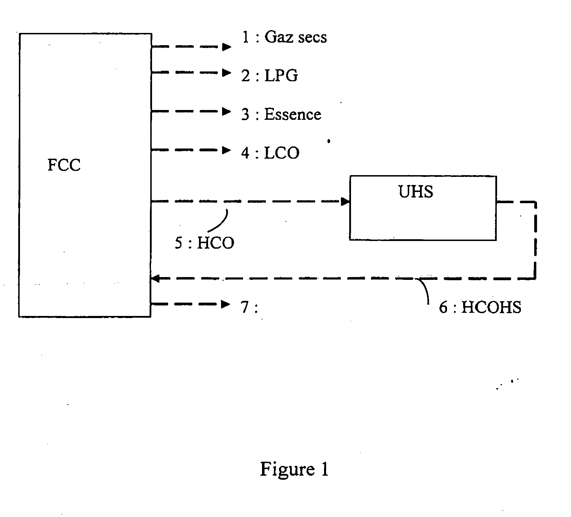

La figure 1 représente un schéma du procédé selon l'invention, dans lequel on a représenté l'unité de craquage catalytique (FCC) de laquelle on extrait le flux de gaz secs (1), le flux de LPG (2), le flux d'essence (3), le flux de coupe LCO (4) et le flux (5) de coupe (HCO) qui est envoyé à l'unité d'hydrogénation sélective (UHS). Ce flux hydrogéné sélectivement (6) et noté HCOHS est recyclé à l'unité de FCC (flux 6). Le flux (7) représente la coupe dite "slurry", c'est à dire une coupe 440°C+.

### DESCRIPTION SOMMAIRE DE L'INVENTION

L'invention concerne un procédé de conversion d'une charge hydrocarbonée dite lourde, c'est à dire constituée d'hydrocarbures de température d'ébullition supérieure à environ 340°C, en vue d'améliorer la production de distillat moyen et de réduire la production d'essence.

On entend par distillat moyen, noté LCO, une coupe d'intervalle de distillation compris entre 220°C et 360°C.

On entend par essence la coupe d'intervalle de distillation 70°C à 150°C.

Le procédé selon l'invention comprend au moins deux étapes réactionnelles, une première étape de craquage catalytique traitant une charge hydrocarbonée lourde telle qu'un distillat sous vide ou un résidu atmosphérique, voire même dans certains cas un résidu sous vide,
et une seconde étape d'hydrogénation sélective de la coupe distillat lourd résultante du FCC, notée HCO, seule ou en mélange avec toute autre coupe riche en composés triaromatiques provenant par exemple des unités de viscoréduction, de Coker, d'unité de type Hoil ou encore la coupe Pygas de l'unité de vapocraquage.

Le caractère sélectif de l'hydrogénation de la coupe distillat lourd (HCO) permet de limiter la formation de monoaromatiques qui augmentent la production de la coupe essence après craquage dans le riser du FCC, la coupe essence n'étant pas recherchée dans le mode de fonctionnement maxi LCO qui est précisément la marche faisant l'objet de la présente invention.

La transformation des triaromatiques permet de produire des diaromatiques indispensables à la production de LCO, mais aussi de limiter la formation de coke, produit majeur de ces composés après passage dans le FCC. Au final, l'hydrogénation sélective de la coupe distillat lourd (HCO) permet d'améliorer nettement la sélectivité distillat moyen (LCO) sur essence par rapport à un recycle de cette coupe au FCC sans hydrotraitement ou avec un hydrotraitement conventionnel.

Dans la suite du texte les termes "hydrotraitement" et "hydrogénation sélective" sont à considérer comme synonymes. On parlera donc indifféremment de coupe HCO hydrotraitée ou de coupe HCO hydrogénée sélectivement.

Le procédé selon l'invention permet de répondre à deux objectifs :
- valoriser la coupe distillat lourd (HCO) ou toute coupe riche en triaromatiques en limitant la production de coke additionnel
- augmenter la production de distillat moyen (LCO) en même temps que la sélectivité distillat moyen sur essence.

La coupe distillat moyen (LCO) correspond à une coupe hydrocarbonée d'intervalle de distillation compris entre 220°C et 360°C.

Le premier objectif de valorisation de la coupe distillat lourd (HCO) produit au sein du FCC ou de toute coupe riche en triaromatiques est assuré en envoyant cette coupe à une ou plusieurs unités d'hydrogénation afin de réduire sa teneur en triaromatiques précurseurs de coke et de composés lourds non valorisables après recycle dans la zone réactionnelle du FCC.

Le deuxième objectif d'amélioration de la production de distillat moyen (LCO) et de la sélectivité distillat moyen sur essence est obtenu en déterminant finement les conditions opératoires de l'hydrogénation sélective de la coupe HCO ex FCC afin de transformer sélectivement les composés triaromatiques en diaromatiques, précurseurs de distillat moyen, tout en minimisant la production de monoaromatiques, qui sont des précurseurs d'essence.

La charge hydrocarbonée lourde est craquée dans un réacteur de craquage catalytique en lit fluidisé en présence d'un catalyseur de craquage.

La coupe distillat lourd (HCO) ou tout autre coupe riche en composés triaromatiques est hydrotraitée sélectivement en présence d'un catalyseur d'hydrotraitement composé d'un ou plusieurs métaux du groupe VIB, préférentiellement le molybdène ou le tungstène, le plus souvent associé avec un ou plusieurs métaux du groupe VIII, préférentiellement le nickel ou le cobalt, et déposé sur un support minéral amorphe, préférentiellement l'alumine, la silice, la silice-alumine, la magnésie, les argiles et les mélanges d'au moins deux de ces éléments.

Le support peut également renfermer d'autres composés tels que par exemple des oxydes choisis dans le groupe formé par l'oxyde de bore, la zircone, l'oxyde de titane ou l'anhydride phosphorique. Le catalyseur peut être frais, partiellement coké ou régénéré.

On peut par exemple employer un catalyseur comprenant 1 à 10 % en poids de nickel et de préférence de 1 à 5 % en poids de nickel (exprimé en oxyde de nickel NiO) associé à 1 à 30 % en poids de molybdène et de préférence 5 à 20 % en poids de molybdène (exprimé en oxyde de molybdène MoO₃) sur un support alumine.

La fraction distillat lourd hydrotraité issue de l'hydrogénation sélective est craquée avec le même catalyseur de craquage, séparément de la charge hydrocarbonée lourde.

Les effluents du craquage catalytique des deux charges sont envoyés dans une zone de fractionnement commune, et le catalyseur utilisé pour le craquage des deux charges est régénéré dans une zone de régénération commune.

Comme exposé dans le paragraphe suivant, l'unité de craquage catalytique comporte. deux réacteurs traitant l'un la charge hydrocarbonée lourde, et l'autre la charge distillat lourd hydrotraitéé sélectivement (HCO hydrotraité).

De plus, chaque réacteur fonctionne en écoulement ascendant

Dans le cas de plusieurs unités d'hydrotraitement sélectif associées à l'unité de craquage catalytique, ces dernières pourront être agencées en série ou en parallèle.

### DESCRIPTION DETAILLEE DE L'INVENTION

Selon l'invention, la charge globale à craquer contient plus de 50 % poids d'hydrocarbures ayant un point d'ébullition supérieur à 340°C. Généralement la charge hydrocarbonée lourde traitée au FCC est constituée d'un distillat sous vide, ou éventuellement d'un résidu atmosphérique. La charge globale craquée peut contenir jusqu'à 100 % poids d'hydrocarbures ayant un point d'ébullition supérieur à 340°C.

Selon l'invention, le catalyseur de craquage est constitué d'une matrice d'alumine, de silice ou de silice alumine avec ou sans une zéolithe de type Y ultra stable dispersée dans cette même matrice. L'ajout d'un additif à base de zéolithe ZSM5, la quantité en cristaux de ZSM5 dans l'inventaire total de l'unité de craquage étant inférieure à 30% poids peut également être envisagé.

Le catalyseur de l'unité d'hydrogénation sélective est un catalyseur comprenant 1 % à 10 % en poids de nickel et de préférence de 1 % à 5 % en poids de nickel (exprimé en oxyde de nickel NiO) associé à 1 % à 30 % en poids de molybdène, et de préférence 5% à 20 % en poids de molybdène (exprimé en oxyde de molybdène MoO₃) sur un support alumine.

L'invention peut donc se définir comme un procédé permettant la valorisation d'une coupe distillat lourd produite au sein du FCC ou de toute autre coupe riche en triaromatiques non valorisables, en augmentant la production de la coupe distillat moyen après recyclage dans la zone réactionnelle du FCC tout en améliorant la sélectivité distillat moyen sur essence et en limitant la production de coke additionnel associé au recycle de ces coupes riches en triaromatiques.

Le procédé selon la présente invention fait appel à une unité de craquage catalytique suivie d'une ou plusieurs unités d'hydrogénation sélectives, procédé dans lequel la charge de l'unité d'hydrogénation sélective est constituée d'une coupe distillat lourd issue du FCC (notée HCO), coupe principalement constituée de triaromatiques à plus de 60%, et caractérisé par un intervalle de distillation compris entre 320°C et 490°C préférentiellement 360°C-440°C, cette coupe (LCO) étant traitée seule ou en mélange avec toute coupes riches en triaromatiques, provenant par exemple des unités de viscoréduction, de Coker, d'Hoil ou encore la coupe Pygas de l'unité de vapocraquage.

Grâce à des conditions opératoires optimisées au niveau de l'hydrogénation sélective, les effluents de l'unité d'hydrogénation sélective présentent une sélectivité diaromatiques sur monoaromatiques optimisée et une proportion de triaromatiques limités. La minimisation des monoaromatiques permet de limiter la formation d'essence lors du craquage de cet effluent dans l'unité de craquage catalytique, la transformation des triaromatiques en diaromatiques permet quant à elle de maximiser la production de la coupe distillat moyen dans l'unité de FCC.

Enfin, la minimisation des composés triaromatiques limite la formation de coke additionnel lié au craquage de recycle de distillat lourd et par conséquent impacte peu le bilan thermique de l'unité de craquage catalytique.

L'unité de craquage catalytique comporte deux réacteurs distincts traitant l'un la charge lourde, l'autre l'effluent de l'unité d'hydrogénation sélective. De plus, chacun des réacteurs est à écoulement ascendant.

Le craquage catalytique est effectué dans deux réacteurs FCC distincts à écoulement ascendant, le premier réacteur FCC effectuant le craquage de la charge lourde travaille à une température de sortie réacteur (ROT1) comprise entre 450°C et 650°C, de préférence comprise entre 470°C et 620°C, et un rapport C/O compris entre 2 et 20, préférentiellement entre 4 et 15. Le second réacteur FCC effectuant le craquage de l'effluent de l'hydrogénation sélective, c'est à dire la coupe distillat lourd hydrotraitée ( HCO) travaille à une température de sortie réacteur (ROT2) comprise entre 500°C et 600°C, préférentiellement comprise entre 520°C et 580°C, avec un rapport C/O compris entre 2 et 20.

Les flux de catalyseur usé issus des deux réacteurs FCC sont séparés des effluents de craquage par tout système de séparation gaz solide connu de l'homme du métier et régénérés dans une zone de régénération commune.

Les deux effluents sont envoyés dans une zone de fractionnement pour produire plusieurs coupes dont une coupe distillat lourd d'intervalle de distillation compris entre 320°C et 490°C préférentiellement 360°C-440°C qui est envoyée ensuite à l'unité d'hydrogénation sélective.

L'unité d'hydrogénation sélective est opérée à une pression entre 15 et 100 bars d'hydrogène, préférentiellement 15 et 50 bar et à une température entre 310°C et 400°C préférentiellement entre 325°C et 360°C en présence d'un catalyseur d'hydrotraitement

Les conditions particulières des différentes étapes du procédé selon l'invention sont décrites ci-après plus en détail.

### 1) Craquage catalytique (FCC) :

Le catalyseur des réacteurs FCC est typiquement constitué de particules de diamètre moyen généralement compris entre 40 et 140 micromètres, et le plus souvent compris entre 50 et 120 micromètres.

Le catalyseur de craquage catalytique contient au moins une matrice appropriée telle que l'alumine, la silice ou la silice-alumine avec présence ou non d'une zéolithe de type Y dispersée dans cette matrice.

Le catalyseur peut comprendre en outre au moins une zéolithe présentant une sélectivité de forme de l'un des types structuraux suivants : MEL (par exemple ZSM-11), MFI (par exemple ZSM-5), NES, EUO, FER, CHA (par exemple SAPO-34), MFS, MWW. Il peut également comprendre l'une des zéolithes suivantes: NU-85, NU-86, NU-88 et IM-5, qui présentent également une sélectivité de forme. L'avantage de ces zéolithes présentant une sélectivité de forme est l'obtention d'une meilleure sélectivité propylène / isobutène, c'est à dire un rapport propylène / isobutène plus élevé dans les effluents de craquage.

La proportion de zéolithe présentant une sélectivité de forme par rapport à la quantité totale de zéolithe peut varier en fonction des charges utilisées et de la structure des produits recherchés. Souvent, on utilise de 0,1% à 60 %, préférentiellement de 0,1% à 40 %, et en particulier de 0,1% à 30 % poids de zéolithe présentant une sélectivité de forme.

La ou les zéolithes peuvent être dispersées dans une matrice à base de silice, d'alumine ou de silice alumine, la proportion de zéolithe (toutes zéolithes confondues) par rapport au poids du catalyseur étant souvent comprise entre 0,7% et 80% poids, de préférence entre 1% et 50% poids, et de manière encore préférée entre 5% et 40 % poids.

Dans le cas ou plusieurs zéolithes sont utilisées, elles peuvent être incorporées dans une seule matrice ou dans plusieurs matrices différentes. La teneur en zéolithe présentant une sélectivité de forme dans l'inventaire totale est inférieure à 30% poids.

Le catalyseur utilisé dans les réacteurs de craquage catalytique peut être constitué d'une zéolithe de type Y ultra stable dispersée dans une matrice d'alumine, de silice, ou de silice alumine, à laquelle on ajoute un additif à base de zéolithe ZSM5, la quantité en cristaux de ZSM5 dans l'inventaire total étant inférieure à 30% poids. L'unité de séparation des effluents des réacteurs de craquage catalytique (FCC) comporte généralement une séparation primaire des effluents du FCC permettant entre autre la production des coupes distillat moyen et distillat lourd.

### 2) Hydrogénation sélective

L'objet de cette étape est d'hydrogéner sélectivement la coupe distillat lourd produite dans l'unité de craquage catalytique ou toute autre coupe présentant une forte teneur en triaromatiques provenant par exemple des unités de viscoréduction, de Coker, de type Hoil ou encore la coupe Pygas de l'unité de vapocraquage. L'optimisation fine des conditions opératoires de l'unité d'hydrogénation sélective permet de transformer majoritairement les composés triaromatiques en diaromatiques en limitant la formation de monoaromatiques. Grâce à ces conditions opératoires optimisées, l'effluent résultant présente un potentiel de production de distillat moyen accru et proportionnel à sa teneur en diaromatiques en même temps qu'un potentiel de coke additionnel et d'essence limité respectivement grâce à la conversion des composés triaromatiques et à la limitation des composés monoaromatiques.

L'hydrogénation sélective peut être réalisée en une ou plusieurs étapes, avec un ou plusieurs réacteurs agencés en parallèle ou en série, et un ou plusieurs catalyseurs. La description suivante du catalyseur et des conditions opératoires peut s'appliquer à l'une quelconque des étapes et/ou à l'un quelconque des réacteurs.

On emploie un catalyseur comprenant 1 % à 10 % en poids de nickel et de préférence de 1 % à 5 % en poids de nickel (exprimé en oxyde de nickel NiO) associé à 1% à 30 % en poids de molybdène et de préférence 5% à 20 % en poids de molybdène (exprimé en oxyde de molybdène MoO₃) sur un support alumine.

La température opératoire de l'hydrogénation sélective est comprise entre 310 et 400°C, et préférentiellement comprise entre 325°C et 360°C.

La pression opératoire de l'hydrogénation sélective est comprise entre 15 bars et 100 bars et préférentiellement 15 bars et 50 bars ( 1 bar = 10 puissance 5 Pascal) L'invention est explicitée plus en détail au moyen de la description de la figure 1.

### EXEMPLES

On fournit ci dessous 3 exemples pour illustrer les performances améliorées du procédé par rapport aux procédés selon l'art antérieur.

### Exemple 1 (art antérieur) : Cas de référence

Le cas de référence traite d'un recycle direct d'HCO sans hydrotraitement au sein du FCC dans un riser dédié.

La composition du HCO issu du craquage d'une charge lourde conventionnelle au FCC est présentée dans le Tableau 1 ("aro" est l'abréviation d'aromatiques)

**Tableau 1 : Composition du HCO ex FCC**

| | monoaro | diaro | triaro + | Di/mono | SPGR |
|---|---|---|---|---|---|
| HCO ex FCC | 8,1 | 7,7 | 84,1 | 0,949 | 0,957 |

Cette coupe HCO est principalement composée d'espèces aromatiques dont 84% poids de triaromatiques. Le ratio diaromatiques sur monoaromatiques est relativement faible et se situe autour de 1.

Cette coupe HCO, soumis aux conditions de craquage d'un riser de FCC, génère les produits présentés dans le Tableau 2. Les rendements indiqués correspondent à des rendements poids par rapport à la charge HCO.

**Tableau 2 : Structure de rendement après craquage direct du HCO au FCC**

| | HCO |
|---|---|
| Gaz secs | 1,8 |
| LPG | 6,0 |
| LCN | 11,5 |
| HCN | 5,6 |
| Essence totale | 17,1 |
| LCO | 5,6 |
| HCO | 63,8 |
| "Slurry" | 2,7 |
| Coke | 3,1 |
| LCO/essence | 0,32 |

Ce recycle direct d'HCO permet ainsi de produire 5,6 points additionnel de distillat moyen et présente une sélectivité distillat moyen (LCO) par rapport à l'essence de 0,32.

### Exemple 2 (art antérieur) : Hydrogénation non sélective du HCO

L'exemple 2 considère le cas où l'HCO issu de l'unité de FCC est envoyé à une unité d'hydrogénation conventionnelle dans les conditions opératoires indiquées dans le Tableau 3

**Tableau 3 : Conditions opératoires de l'hydrogénation non sélective**

| Pression | Température | VVh |
|---|---|---|
| bar | °C | h-1 |
| 70 | 335 | 0,5 |

Dans ces conditions, la coupe résultant de cet hydrotraitement non sélectif présente une composition telle qu'indiquée dans le Tableau 4 ci dessous ( "aro" est l'abréviation d'aromatiques).

**Tableau 4 : Composition de l'HCO après hydrogénation non sélective**

| | monoaro | diaro | triaro + | Total "aro" | Di/mono | SPGR |
|---|---|---|---|---|---|---|
| HDT non sélective d'HCO | 24,4 | 39,3 | 36,3 | 100 | 1,61 | 0,9831 |

L'hydrotraitement non sélectif permet de transformer une large partie des triaromatiques en di et mono aromatiques. Même si la quantité en diaromatiques augmente largement par rapport au cas sans hydrogénation, cette augmentation s'accompagne d'une forte progression des monoaromatiques.

Craqué dans un riser de FCC dans les mêmes conditions opératoires que l'exemple 1, cette coupe HCO hydrotraitée de manière non sélective conduit à 8 points de distillat moyen, soit une augmentation de plus de 2 points par rapport au cas sans hydrotraitement, ce qui est très significatif au regard de tonnages mis en jeu dans le procédé FCC.

Toutefois, cette augmentation s'accompagne également d'une forte progression de l'essence totale qui double quasiment du fait de la forte proportion en monoaromatiques de l'HCO hydrotraité non sélectivement. Ainsi la sélectivité distillat moyen par rapport à l'essence chute fortement à 0,25.

Dans un contexte fort de minimisation de la production d'essence, le gain obtenu en distillat moyen ne compense pas la pénalité résultant de l'augmentation de la production d'essence.

**Tableau 5 : Structure de rendement après craquage direct du HCO hydrotraité non sélectivement au FCC en comparaison avec le cas de référence**

| | HCO | HDT non sélective de l'HCO |
|---|---|---|
| Gaz secs | 1,8 | 1,5 |
| LPG | 6,0 | 7,8 |
| LCN | 11,5 | 21,7 |
| HCN | 5,6 | 10,0 |
| Total essence | 17,1 | 31,6 |
| LCO | 5,6 | 8,0 |
| HCO | 63,8 | 47,7 |
| "Slurry" | 2,7 | 2,0 |
| Coke | 3,1 | 1,4 |
| LCO/essence | 0,32 | 0,25 |

### Exemple 3 (selon l'invention) : Hydrogénation sélective de l'HCO

L'exemple 3 correspond à l'invention. Le HCO résultant de l'unité de FCC est envoyé vers une unité d'hydrogénation sélective pour laquelle les conditions opératoires ont été fixées de manière fine afin de transformer les triaromatiques en diaromatiques tout en limitant la production de monoaromatiques. Les conditions opératoires considérées dans notre étude sont indiquées dans le Tableau 6 ci dessous:

**Tableau 6 : Conditions opératoires de l'hydrogénation sélective**

| Pression | Température | VVh | S effluent |
|---|---|---|---|
| bar | °C | h-1 | wt% |
| 25 | 335 | 0,5 | 0,255 |

Il en résulte une composition d'HCO après hydrogénation sélective différente de l'exemple 1. cette composition est présentée dans le Tableau 7 ci dessous ( "aro" est l'abréviation d'aromatiques).

**Tableau 7 : Composition de l'HCO après hydrogénation sélective**

| | monoaro | diaro | triaro + | Total "aro" | Di/mono | SPGR |
|---|---|---|---|---|---|---|
| Select HDT HCO | 8,6 | 21,9 | 69,5 | 100 | 2,55 | 0,9818 |

Dans les condition d'hydrogénation sélective, la proportion de diaromatiques est certes légèrement plus faible que dans le cas de l'exemple 2, mais la quantité de monoaromatiques est fortement limitée. Il en résulte que le ratio diaromatiques sur monoaromatiques est nettement plus favorable à 2,55 contre 1,6 dans le cas d'une hydrogénation non sélective.

On peut donc s'attendre à une nette amélioration de la sélectivité en distillat moyen par rapport à l'essence après craquage au FCC.

Les rendements obtenus après craquage de l'HCO hydrogéné sélectivement sont présentés dans le Tableau 8 ci dessous et comparés à ceux obtenus dans le cas d'une hydrogénation non sélective et du craquage direct.

**Tableau 8 : Structure de rendement après craquage direct du HCO hydrotraité sélectivement au FCC en comparaison avec le cas de référence et l'exemple1**

| | HCO | HDT non sélective de l'HCO | HDT sélective de l'HCO |
|---|---|---|---|
| Gaz secs | 1,8 | 1,5 | 1,5 |
| LPG | 6,0 | 7,8 | 5,7 |
| LCN | 11,5 | 21,7 | 13,5 |
| HCN | 5,6 | 10,0 | 7,1 |
| Total essence | 17,1 | 31,6 | 20,5 |
| LCO | 5,6 | 8,0 | 7,3 |
| HCO | 63,8 | 47,7 | 60,5 |
| "Slurry" | 2,7 | 2,0 | 2,4 |
| Coke | 3,1 | 1,4 | 2,0 |
| LCO/essence | 0,32 | 0,25 | 0,36 |

Comme attendu, la sélectivité distillat moyen sur essence est très fortement amélioré à 0,36 lorsque l'HCO est hydrogéné sélectivement. Cette sélectivité est même meilleure que celle obtenue dans le cas d'un recycle direct d'HCO sans hydrotraitement.

Le gain en distillat moyen dans le cas de l'hydrotraitement sélectif de la coupe HCO est plus élevé que dans le cas d'un recycle direct de cette même coupe 7,3 contre 5,6 et légèrement plus faible que dans le cas d'un hydrotraitement non sélectif de la coupe HCO.

L'avantage majeur de l'hydrotraitement sélectif se situe au niveau du rendement de la coupe essence qui reste limitée à environ 20%, et se trouve finalement très proche du rendement obtenu dans le cas d'un recycle direct de la coupe HCO.

Ce rendement en coupe essence est également nettement plus faible que dans le cas d'une hydrogénation non sélective de la coupe HCO.

Comme pour l'hydrotraitement non sélectif, le coke issu du craquage d'un HCO hydrogéné sélectivement reste limité et plus faible que dans le cas d'un recycle direct de la coupe HCO, ce qui permet de ne pas perturber le bilan thermique de l'unité par l'ajout de coke additionnel.

Cet exemple 3 illustre clairement les deux objectifs de l'invention à savoir :
- valoriser la coupe distillat lourd ou toute coupe riche en triaromatiques en limitant la production de coke additionnel
- augmenter la production de distillat moyen en même temps que la sélectivité distillat moyen sur essence.

## Revendications

1. Procédé de conversion d'une charge hydrocarbonée lourde présentant une sélectivité améliorée en distillat moyen (LCO) d'intervalle de distillation compris entre 220°C et 360°C, ledit procédé faisant appel à une unité de craquage catalytique (FCC) suivie d'une ou plusieurs unités d'hydrogénation sélectives, procédé dans lequel la charge de l'unité d'hydrogénation sélective est constituée d'une coupe distillat lourd issue du FCC dite coupe (HCO), constituée de triaromatiques à plus de 60% poids, et **caractérisé par** un intervalle de distillation compris entre 320°C et 490°C, préférentiellement 360°C et 440°C, ladite coupe HCO hydrogénée sélectivement étant réintroduite dans la zone réactionnelle de l'unité FCC, procédé dans lequel l'unité d'hydrogénation sélective est opérée à une pression entre 15 et 50 bar, et à une température comprise entre 325°C et 360°C en présence d'un catalyseur d'hydrotraitement comprenant 1 à 10 % en poids de nickel et de préférence de 1 à 5 % en poids de nickel (exprimé en oxyde de nickel NiO) associé à 1 à 30 % en poids de molybdène et de préférence 5 à 20 % en poids de molybdène (exprimé en oxyde de molybdène MoO₃) sur un support alumine procédé dans lequel l'unité de craquage catalytique comporte deux réacteurs ascendants distincts,
- le premier traitant la charge hydrocarbonée lourde aux conditions suivantes: température de sortie réacteur (ROT1) comprise entre 450°C et 650°C, de préférence comprise entre 470°C et 620°C, et un rapport C/O compris entre 2 et 20, préférentiellement entre 4 et 15,
- et le second traitant la coupe distillat lourd hydrotraitée (HCO) aux conditions suivantes: température de sortie réacteur (ROT2) comprise entre 500°C et 600°C, préférentiellement comprise entre 520°C et 580°C, avec un rapport C/O compris entre 2 et 20.

2. Procédé de conversion d'une charge hydrocarbonée lourde présentant une sélectivité améliorée en distillat moyen (LCO) selon la revendication 1, dans lequel la coupe (HCO) issue de l'unité FCC est introduite dans l'unité d'hydrogénation sélective en mélange avec toute coupe riche en tri aromatiques, telles que les coupes provenant des unités de viscoréduction, de Coker, d'unité de type Hoil ou encore la coupe Pygas issue d'une unité de vapocraquage.

## Patentansprüche

1. Verfahren zur Umwandlung einer schweren Kohlenwasserstoffcharge, das eine verbesserte Selektivität für Mitteldestillat (LCO) mit einem Destillationsintervall im Bereich zwischen 220°C und 360°C aufweist, wobei das Verfahren eine Einheit zum katalytischen Cracken (FCC), gefolgt von einer oder mehreren Einheiten zur selektiven Hydrierung verwendet, bei dem die Charge der Einheit zur selektiven Hydrierung aus einem Schnitt eines aus der FCC stammenden schweren Destillats, genannt Schnitt (HCO) besteht, der aus Triaromaten mit mehr als 60 Gew.-% besteht, und durch ein Destillationsintervall im Bereich zwischen 320°C und 490°C, bevorzugt 360 °C und 440 °C, gekennzeichnet ist, wobei der selektiv hydrierte HCO-Schnitt wieder in die Reaktionszone der FCC-Einheit eingeführt wird, bei dem die Einheit zur selektiven Hydrierung bei einem Druck zwischen 15 und 50 bar und einer Temperatur im Bereich zwischen 325°C und 360°C in Gegenwart eines Katalysators zur Hydrobehandlung betrieben wird, der 1 bis 10 Gew.-% Nickel und bevorzugt 1 bis 5 Gew.-% Nickel (ausgedrückt in Nickeloxid NiO), kombiniert mit 1 bis 30 Gew.-% Molybdän und bevorzugt 5 bis 20 Gew.-% Molybdän (ausgedrückt in Molybdänoxid MoO₃) auf einem Aluminiumoxidträger umfasst, bei dem die Einheit zum katalytischen Cracken zwei verschiedene Riser-Reaktoren umfasst,
- wobei der erste die schwere Kohlenwasserstoffcharge unter folgenden Bedingungen behandelt: Reaktorausgangstemperatur (ROT1) im Bereich zwischen 450°C und 650°C, bevorzugt im Bereich zwischen 470°C und 620°C, und ein C/O-Verhältnis im Bereich zwischen 2 und 20, bevorzugt zwischen 4 und 15,
- und der zweite den Schnitt aus dem schweren hydrobehandelten Destillat (HCO) unter folgenden Bedingungen behandelt: Reaktorausgangstemperatur (ROT2) im Bereich zwischen 500°C und 600°C, bevorzugt im Bereich zwischen 520°C und 580°C, mit einem C/O-Verhältnis im Bereich zwischen 2 und 20.

2. Verfahren zur Umwandlung einer schweren Kohlenwasserstoffcharge, das eine verbesserte Selektivität für Mitteldestillat (LCO) aufweist, nach Anspruch 1, in dem der aus der FCC-Einheit stammende Schnitt (HCO) als Gemisch mit jedem Schnitt, der reich an Triaromaten ist, wie etwa die Schnitte, die aus Visbreaking-Einheiten, Koker-Einheiten, einer Einheit des HOil-Typs stammen, oder auch der Pygas-Schnitt, der aus einer Einheit zum Dampfcracken stammt, wieder in die Einheit zur selektiven Hydrierung eingeführt wird.

## Claims

1. A process for converting a heavy hydrocarbon feedstock having improved selectivity for middle distillate (LCO) with a distillation range in the range 220°C to 360°C, said process using a catalytic cracking unit (FCC) followed by one or more selective hydrogenation units, in which process the feedstock for the selective hydrogenation unit is constituted by a heavy distillate cut obtained from FCC of said cut (HCO), constituted by triaromatics in an amount of more than 60% by weight, and **characterized by** a distillation range in the range 320°C to 490°C, preferably 360°C to 440°C, said selectively hydrogenated HCO cut being re-introduced into the reaction zone of the FCC unit, in which process the selective hydrogenation unit is operated at a pressure in the range 15 to 50 bars, at a temperature in the range 325°C to 360°C, using a catalyst comprising 1% to 10% by weight of nickel, preferably 1% to 5% by weight of nickel (expressed as nickel oxide, NiO) associated with 1% to 30% by weight of molybdenum, preferably 5% to 20% by weight of molybdenum (expressed as molybdenum oxide, MoO₃) on an alumina support, said process in which the catalytic cracking unit comprises two distinct upflow reactors:
• the first processing the heavy hydrocarbon feedstock under the following conditions: reactor outlet temperature (ROT1) in the range 450°C to 650°C, preferably in the range 470°C to 620°C, and a C/O ratio in the range 2 to 20, preferably in the range 4 to 15;
• and the second processing the hydrotreated heavy distillate cut (HCO) under the following conditions: reactor outlet temperature (ROT2) in the range 500°C to 600°C, preferably in the range 520°C to 580°C, and a C/O ratio in the range 2 to 20.

2. A process for converting a heavy hydrocarbon feedstock having improved selectivity for middle distillate (LCO) according to claim 1, in which the (HCO) cut obtained from the FCC unit is introduced into the selective hydrogenation unit as a mixture with any cut which is rich in triaromatics, such as cuts from visbreaking, coking, "H-oil" type units or the Pygas cut from a steam cracking unit.
